# EUROPEAN PATENT APPLICATION

(11) **EP 4 574 051 A1**
(43) Date of publication of application: **25.06.2025**
(21) Application number: 23307308.9
(22) Date of filing: 21.12.2023
(51) Int. Cl.: A61B 8/04, A61B 8/08, A61B 5/02, A61B 8/12

(54) **ANEURYSMAL STATE DETECTION**

(71) Applicant: Koninklijke Philips N.V., 5656 AG Eindhoven (NL)
(72) Inventor: BRACCO, Marta Irene, 5656 Eindhoven (NL); ROUET, Laurence, 5656 Eindhoven (NL); AVRIL, Stéphane, 5656 Eindhoven (NL); EIBERG, Jonas Peter, 5656 Eindhoven (NL)
(74) Representative: Philips Intellectual Property & Standards

(57) **Abstract**

An ultrasound system (100) is configured to: determine, based on blood pressure measurements and changes in response of vasculature wall tissue between two-dimensional ultrasound image sequences as pressure is variably applied, a non-linear elastic relationship between strains in vasculature wall tissue captured in the two-dimensional ultrasound image sequences and varying stresses to which the vasculature wall tissue is subjected based on subject-specific blood pressure. The ultrasound system (100) is also configured to detect an aneurysmal state of the vasculature wall tissue based on the non-linear elastic relationship and the changes in response of the vasculature wall tissue between the two-dimensional ultrasound image sequences as the pressure is variably applied.

## Description

### FIELD OF THE INVENTION

The invention relates to aneurysmal state detection of vasculature wall tissue, and to a computer program product and an ultrasound system to do so.

### BACKGROUND OF THE INVENTION

An abdominal aortic aneurysm (abdominal aortic aneurysm) is a permanent dilation of a portion of the abdominal aorta, commonly the infrarenal artery. With an estimated mortality rate of 60 to 80% after rupture, abdominal aortic aneurysms are a leading cause of death in men over 65. The typical bulge-like formation is caused by a progressive degradation of the aortic wall, which results in a loss of elasticity and general weakening of the affected portion of the artery. Early diagnosis of abdominal aortic aneurysm is possible thanks to current imaging techniques, among which abdominal ultrasonography is the most accessible and widespread. Asymptomatic abdominal aortic aneurysm detection allows preventive surgical treatment and reduces the number of rupture events. However, surgery often leads to complications, causing patient morbidity and mortality. Therefore, a need exists to optimize clinical decision-making guidelines by predicting rupture in the most effective way.

In current practice, decisions are based on the maximum diameter criterion, which sets a threshold value for the antero-posterior diameter above which surgery is advised. The threshold value is between 5 and 5.5 centimeters. A criterion based solely on the antero-posterior diameter fails to characterize the entire population. Retrospective studies on abdominal aortic aneurysm ruptures have questioned the validity of the maximum diameter criterion for decades and shown that the probability of rupture is still relatively high for some subjects with small aneurysms below 5 centimeters. The studies have also shown that abdominal aortic aneurysms up to 10 centimeters often dilate without rupturing. Therefore, calls have been made to shift clinical practice towards more subject specific biomarkers that include biomechanical characteristics. One of the most important aspects of abdominal aortic aneurysm wall biomechanics has proven to be the change in stiffness related to the loss of elastic fibers, but as of yet there is inadequate research that would lead to improving abdominal aortic aneurysm management.

Time-resolved two-dimensional ultrasound has been used to assess abdominal aortic aneurysm wall stiffness which otherwise typically involves in vitro mechanical testing on samples acquired intra-operatively or on high fidelity computational models which may lack validation, and which may be difficult to translate in a clinical environment. Two-dimensional ultrasound is the most widespread imaging technique for abdominal aortic aneurysm monitoring and allows to obtain time-resolved (or cine-loop) sequences that have a higher temporal resolution than four-dimensional ultrasounds and cine-MRI techniques. The main direction of the abdominal aortic aneurysm wall cyclic deformation in response to blood pressure is radial, so perpendicular to the abdominal aortic aneurysm centerline.

Abdominal aortic aneurysm walls may also be characterized biomechanically. Biomechanical characterization involves the choice of a constitutive material model, describing the relationship between stresses and strains in the tissue. The cyclic dilation of the aorta, in response to the pulsatile blood pressure, has been harnessed to obtain a tangent modulus of elasticity. Thus, it is common practice to linearize the constitutive equation describing the aneurysm wall within the physiological pressure range, as in the echo-tracking ultrasound system. This tool relates the abdominal aortic aneurysm diameter change to the (brachial) blood pressure and provides a straightforward measurement of the abdominal aortic aneurysm wall elastic modulus, as a function of the difference between systolic and diastolic brachial pressure values, and the difference between systolic and diastolic antero-posterior diameters acquired at the maximum diameter plane. However, this tool suffers from moderate intra-observer and inter-observer variations, and unsatisfactory predicting ability for both short term abdominal aortic aneurysm growth and rupture has been reported.

Pressure from the probe being applied by the sonographer is a source of error in biomechanical characterization. Such probe pressure exerted by the sonographer on the patient abdomen during the scan may result in significant diameter measurement bias. Previous works focusing on material characterization with ultrasound have not addressed the impact of probe pressure. Ignoring such an effect is an unexplored source of uncertainty which can negatively affect both the reproducibility and the interpretability of the biomechanical quantifications.

Additionally, clinically available material characterizations are based on linear elastic models. Wall stiffness measurements would benefit from improvements, and one such improvement may result from discarding the assumption of a linear elastic material for the vascular tissue. The assumption of linearity may result in a wrong estimation of the tissue stiffness. Currently there are no methods available to assess non-invasively the parameters of hyperelastic material models, and the rare methods that have been proposed in research to assess parameters of a simple linear elastic model are not translatable to clinics because they make use of three-dimensional image segmentation and physical simulations.

### SUMMARY OF THE INVENTION

It is, inter alia, an object of the invention to provide an improved aneurysmal state detection of vasculature wall tissue. The invention is defined by the independent claims. The dependent claims define advantageous embodiments.

According to an embodiment of the present disclosure, an ultrasound system includes a memory that stores instructions; and a processor that executes the instructions. When executed by the processor, the instructions cause the ultrasound system to: receive time-resolved two-dimensional ultrasound image sequences of vasculature walls in vasculature wall tissue captured at different times as pressure (e.g. probe pressure) is variably applied to a subject of the two-dimensional ultrasound image sequences; receive blood pressure measurements of the subject; determine changes in response of the vasculature wall tissue between the two-dimensional ultrasound image sequences at the different times as the pressure is variably applied to the subject; determine, based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on a combination of subject-specific blood pressure; and detect an aneurysmal state of the vasculature wall tissue based on the non-linear elastic relationship and the changes in response of the vasculature wall tissue between the two-dimensional ultrasound image sequences as the pressure is variably applied.

According to another embodiment of the present disclosure, a method of operation for an ultrasound system comprising a memory that stores instructions and a processor that executes the instructions, includes receiving time-resolved two-dimensional ultrasound image sequences of vasculature wall tissue captured at different times as pressure is variably applied to a subject of the two-dimensional ultrasound image sequences. The method of operation also includes receiving blood pressure measurements of the subject; determining changes in response of the vasculature wall tissue between the two-dimensional ultrasound image sequences at the different times as the pressure is variably applied to the subject; determining, based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on subject-specific blood pressure; and detecting an aneurysmal state of the vasculature wall tissue based on the non-linear elastic relationship and the changes in the vasculature wall tissue between the two-dimensional ultrasound image sequences as the pressure is variably applied.

According to another embodiment of the present disclosure, a tangible, non-transitory computer-readable medium stores instructions. When executed by a processor, the instructions cause the processor to: receive time-resolved two-dimensional ultrasound image sequences of vasculature wall tissue captured at different times as pressure is variably applied to a subject of the two-dimensional ultrasound image sequences; receive blood pressure measurements of the subject; determine changes in response of the vasculature wall tissue between the two-dimensional ultrasound image sequences at the different times as the pressure is variably applied to the subject; determine, based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on a combination of subject-specific blood pressure and externally applied probe pressure variably applied to the subject; and detect an aneurysmal state of the vasculature wall tissue based on the non-linear elastic relationship and the changes in response of the vasculature wall tissue between the two-dimensional ultrasound image sequences as the pressure is variably applied.

These and other aspects of the invention will be apparent from and elucidated with reference to the embodiments described hereinafter.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 illustrates a system for aneurysmal state detection, in accordance with a representative embodiment.
Fig. 2 illustrates another system for aneurysmal state detection, in accordance with a representative embodiment.
Fig. 3 illustrates another system for aneurysmal state detection, in accordance with a representative embodiment.
Fig. 4 illustrates a method for aneurysmal state detection, in accordance with a representative embodiment.
Fig. 5 illustrates antero-posterior diameter evolution in aneurysmal state detection, in accordance with a representative embodiment.
Fig. 6 illustrates a workflow for aneurysmal state detection, in accordance with a representative embodiment.
Fig. 7 illustrates a relationship between stress and strain in aneurysmal state detection, in accordance with a representative embodiment.
Fig. 8 illustrates antero-posterior diameter changes in three subjects in aneurysmal state detection, in accordance with a representative embodiment.
Fig. 9 illustrates a computer system, on which a method for aneurysmal state detection is implemented, in accordance with another representative embodiment.

### DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third and so on may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a,' 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, two-dimensional ultrasounds may be used to study the wall strain in the abdominal aortic aneurysm wall with a combination of speckle tracking and strain mapping. Aneurysmal tissue may be characterized via probe pressure-calibrated ultrasound imaging. Arterial tissue mechanics may be characterized in vivo by means of two-dimensional ultrasound acquisitions with varying probe pressure. A non-linear elastic relationship between the strains in the tissue and the stresses to which the tissue is subjected may be extracted. The teachings herein satisfy a clinical need for a realistic mechanical characterization of the vascular walls in order to detect the tissue state of degradation due to pathological changes. Updating the wall stiffness measurements to rely on a non-linear hyperelastic material model, the values of tangent stiffness modulus can reflect the external probe pressure, and this may result in more accurate measurements. Hyperelastic material models may be necessary to accurately describe mechanical behavior of blood vessels. The difference between a linear model and a non-linear model may be visualized as a chart where the linear model is represented by a line moving up and to the right, and the non-linear model is represented by a curve with a slope that increases to the right. If the visualization is provided on a chart with blood pressure and probe pressure being represented on the Y axis and diameter being represented on the X axis, many measurements of diameter will be expected to be farther to the right on the non-linear model than on the linear model. Accounting for the non-linearity of mechanical behavior of blood vessels may thus result in improved accuracy when detecting aneurysmal states of vasculature wall tissue.

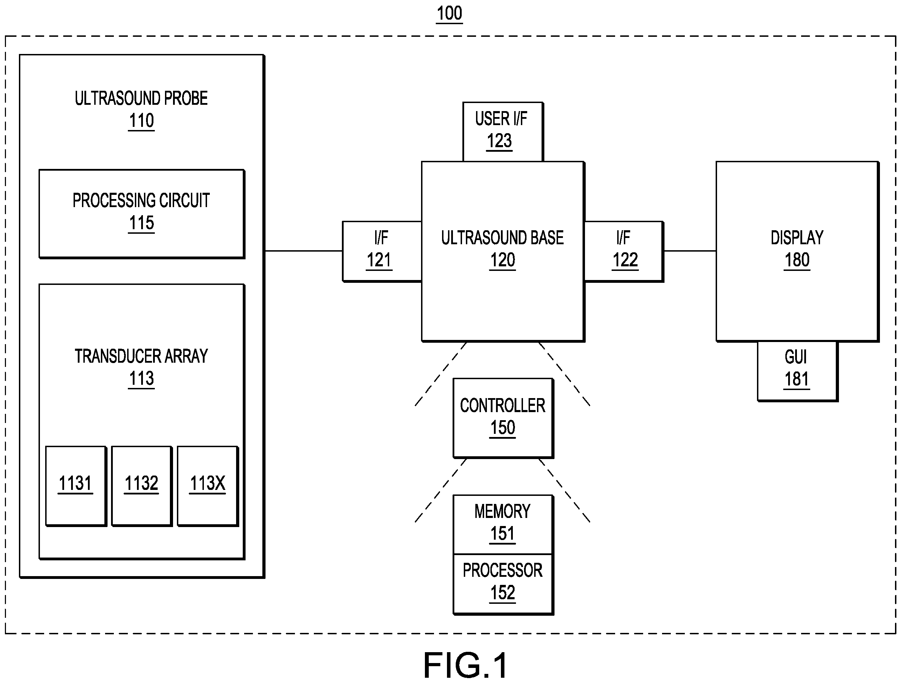

Fig. 1 illustrates an ultrasound system 100 for aneurysmal state detection, in accordance with a representative embodiment.

The ultrasound system 100 in Fig. 1 is a system for aneurysmal state detection and includes components that may be provided together or that may be distributed, an ultrasound probe 110, an ultrasound base 120 and a display 180. The ultrasound system 100 may also be considered an ultrasound processing system that generates and processes ultrasound images. The display 180 includes a graphical user interface 181 (GUI) used to display ultrasound images and related information of the types described herein. Although not shown, the ultrasound system 100 may also include a sensor to measure arterial pressure.

The ultrasound probe 110 includes at least a transducer array 113 and a processing circuit 115. The transducer array 113 includes an array of individual transducer elements including a first transducer element 1131, a second transducer element 1132 through an Xth transducer element 113X. The transducer array 113 may include more than three transducer elements, such as dozens, hundreds or even thousands. The processing circuit 115 may include a combination of a memory that stores instructions and a processor that executes the instructions to, for example, process ultrasound imagery received via the transducer array 113. The processing circuit 115 may also or alternatively include circuit elements of an application-specific integrated circuit (ASIC). The ultrasound base 120 includes a first interface 121, a second interface 122, a third interface 123, and a controller 150. The third interface 123 is a user interface and may be used by users to interact with the ultrasound base 120 such as to enter instructions and receive output. In Fig. 1, the controller 150 includes a memory 151 that stores instructions and a processor 152 that executes the instructions. The controller 150 in Fig. 1 also may include the third interface 123 and may also be considered to include the display 180 as a user interface in some embodiments.

The third interface 123 may allow a sonographer to provide instructions or other input to customize dynamic determination of imaging sequence completeness using the third interface 123 as a user interface. For example, a sonographer may be enabled to set up their own checklist and build customized conditions for completeness. The checklist may then be provided back to the sonographer via the display 180 or the third interface 123 as a user interface to use as a checklist of completed regions of interest as a history of what has been seen.

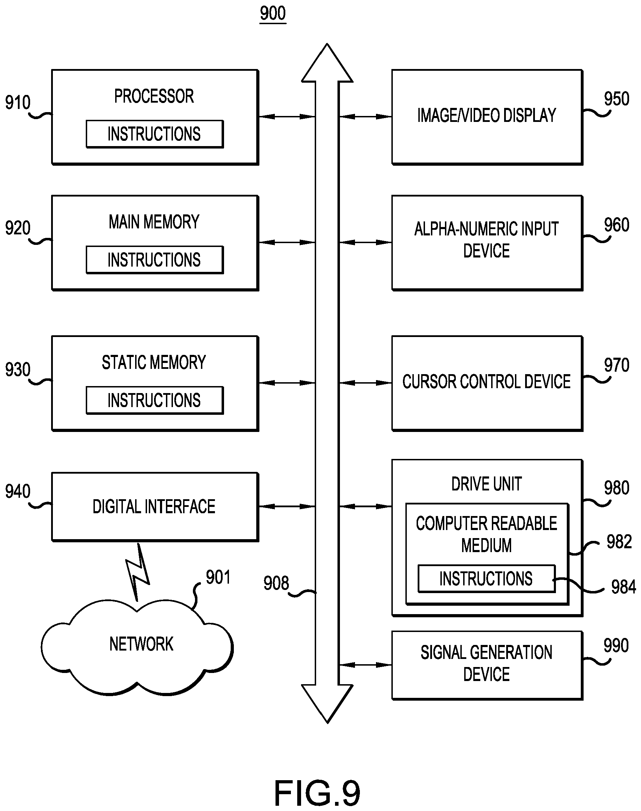

The controller 150 may include a memory 151 that stores instructions and a processor 152 that executes the instructions. A computer that can be used to implement the ultrasound base 120 is depicted in Fig. 9, though an ultrasound base 120 may include more or fewer elements than depicted in Fig. 1 or Fig. 9. In some embodiments, multiple different elements of the ultrasound system 100 in Fig. 1 may include a controller such as the controller 150.

The display 180 may be local to the ultrasound base 120 or may be remotely connected to the ultrasound base 120. The display 180 may be connected to the controller 150 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users.

The controller 150 may perform some of the operations described herein directly and may implement other operations described herein indirectly. For example, the controller 150 may indirectly control operations such as by generating and transmitting content to be displayed on the display 180. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

An aneurysmal state of an abdominal aortic aneurysm may be defined by one or more characteristics as described herein. The aneurysmal state may be quantifiable such as by a score between 1 and 100. The aneurysmal state may reflect the likelihood of rupture of an abdominal aortic aneurysm, such as relative to a specific timeframe. For example, an aneurysmal state may indicate a high likelihood of a rupture in the next day, or a low likelihood of a rupture in the next week. Aneurysmal state may also be indicated as indeterminate, when the aneurysmal state cannot be determined with certainty beyond a predetermined threshold using the teachings herein. In Fig. 1, instructions executed by the processor 152 cause the controller 150 to receive time-resolved two-dimensional ultrasound image sequences of vasculature walls in vasculature wall tissue captured at different times as pressure is variably applied to a subject (e.g., probe pressure) of the two-dimensional ultrasound image sequences. The instructions also cause the controller 150 to receive blood pressure measurements of the subject; determine changes in response of the vasculature wall tissue between the two-dimensional ultrasound image sequences at the different times as the pressure is variably applied to the subject; determine, based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on subject-specific blood pressure; and detect an aneurysmal state of the vasculature wall tissue based on the non-linear elastic relationship and the changes in response of the vasculature wall tissue between the two-dimensional ultrasound image sequences as the pressure is variably applied. The non-linear elastic relationship may also be based on externally applied probe pressure variably applied to the subject and quantified based on measurement.

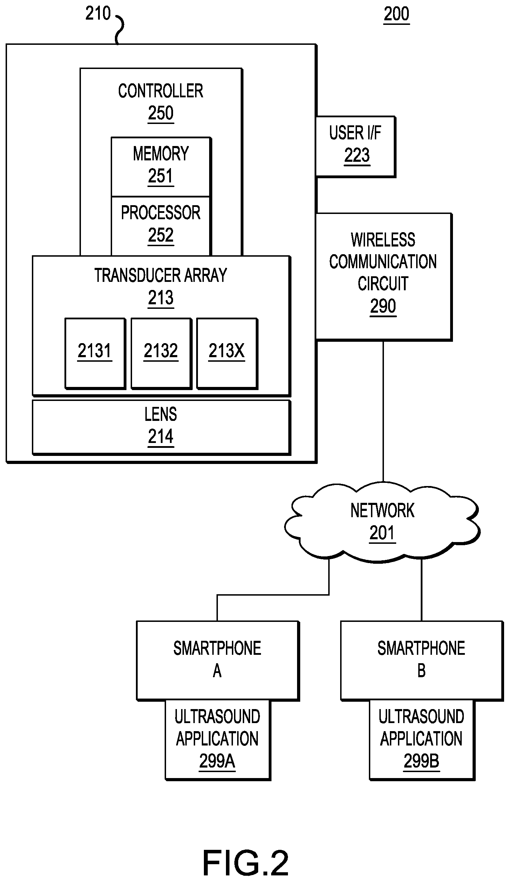

Fig. 2 illustrates another system for aneurysmal state detection, in accordance with a representative embodiment.

The system 200 in Fig. 2 includes an ultrasound system 210. Additionally, while smartphone A and smartphone B are not necessarily considered part of the system 200 in Fig. 2, the ultrasound application 299A on smartphone A and the ultrasound application 299B on smartphone B may be considered part of the system 200 in Fig. 2. Although not shown, the arrangement in Fig. 2 may also include a sensor to measure arterial pressure.

The ultrasound system 210 includes a transducer array 213, a lens 214, a controller 250, a user interface 223 and a wireless communication circuit 290. In Fig. 2, the controller 250 includes a memory 251 that stores instructions and a processor 252 that executes the instructions. The controller 250 may also be considered to include the user interface 223 in some embodiments. In some embodiments, smartphone A and smartphone B may be considered part of the system 200, such as when issued by the same entity that owns or otherwise controls the ultrasound system 210. Smartphone A and smartphone B communicate with the ultrasound system 210 via network 201 and the wireless communication circuit 290. The network 201 may comprise one or more of a wireless local area network (LAN) such as a Wi-Fi network and/or a wired or wireless wide area network (WAN) such as a cellular network and/or the Internet. The ultrasound system 210 may comprise an ultrasound probe such as a portable ultrasound probe that coordinates ultrasound exams with the ultrasound application 299A and/or the ultrasound application 299B.

When executed by the processor 252, the instructions cause the controller 250 to receive time-resolved two-dimensional ultrasound image sequences of vasculature walls in vasculature wall tissue captured at different times as pressure is variably applied to a subject of the two-dimensional ultrasound image sequences; receive blood pressure measurements of the subject; determine changes in response of the vasculature wall tissue between the two-dimensional ultrasound image sequences at the different times as the pressure is variably applied to the subject; determine, based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on subject-specific blood pressure; and detect an aneurysmal state of the vasculature wall tissue based on the non-linear elastic relationship and the changes in response of the vasculature wall tissue between the two-dimensional ultrasound image sequences as the pressure is variably applied. The non-linear elastic relationship may also be based on externally applied probe pressure variably applied to the subject and quantified based on measurement.

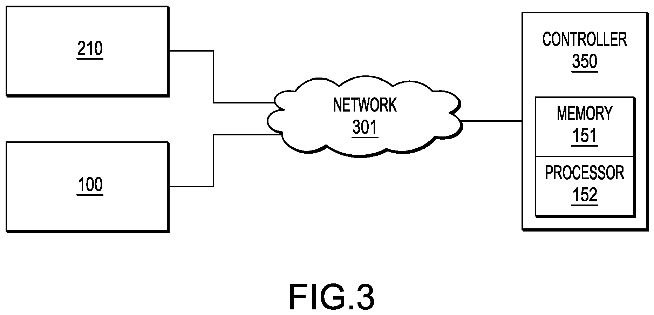

Fig. 3 illustrates another system for aneurysmal state detection, in accordance with a representative embodiment.

The system in Fig. 3 includes the ultrasound system 100 in Fig. 1 and the ultrasound system 210 in Fig. 2, connected to a controller 350 over a network 301. The controller 350 includes the memory 151 that stores instructions and the processor 152 that executes the instructions. The network 301 is representative of a wired and/or wireless wide area network so that the controller 350 may provide cloud-based services to a plurality of systems that perform ultrasound imaging. Aneurysmal state detection may be performed partly or fully in a cloud context provided by the controller 350 as shown in Fig. 3.

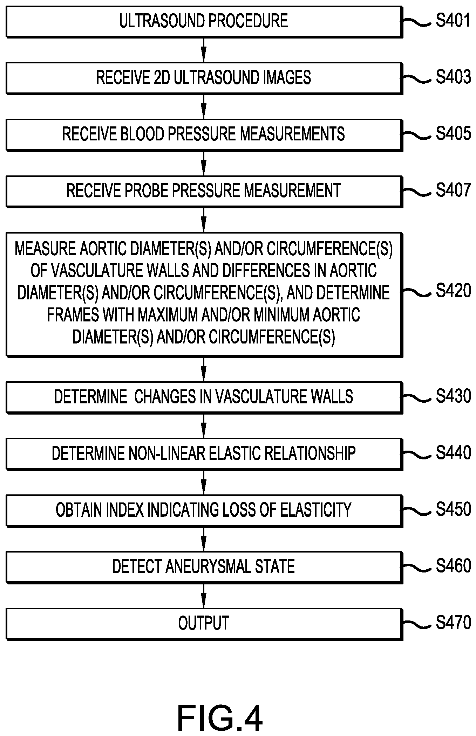

Fig. 4 illustrates a method for aneurysmal state detection, in accordance with a representative embodiment.

The method of Fig. 4 may be performed by the ultrasound system 100 including the controller 150, or by the arrangement in Fig. 2 including the ultrasound system 210 and smartphone A and/or smartphone B.

At S401, an ultrasound procedure is started.

At S403, two-dimensional ultrasound images are received. The two-dimensional ultrasound images may be time-resolved two-dimensional ultrasound image sequences of vasculature walls in vasculature wall tissue captured at different times as pressure is variably applied to a subject of the two-dimensional ultrasound image sequences. The vasculature wall tissue does not itself change in response to pressure. Rather, the responses of the vasculature wall tissue change in response to variable pressure change over time given the varying stresses, and this may be observed by varying amplitudes of deformations due to variable pressures. Accordingly, change in behavior of the vasculature wall tissue is observed under different pressure conditions, and this provides information as to properties of the vasculature wall tissue, The vasculature walls may be aortic walls of the abdominal aorta of the subject. The two-dimensional ultrasound image sequences may each be captured at different times in a single cineloop in some embodiments and may be captured at different times in two separate cineloops in other embodiments. For example, cyclic variations in aortic diameter or circumference may be measured within each of two separate cineloops. As described herein, a main direction of the abdominal aortic aneurysm wall cyclic deformation in response to blood pressure is perpendicular to the abdominal aortic aneurysm centerline. Variations in the diameter may be measured so that cyclic contributions to the variations due to blood pressure can be determined.

At S405, blood pressure measurements are received. The blood pressure measurements may be blood pressure measurements of the subject read by a sensor. For example, the controller 150 in Fig. 1 or smartphone A or smartphone B in Fig. 2 may receive measurements of arterial pressure and determine the non-linear elastic relationship also based on the measurements of the arterial pressure. No particular timing of the two-dimensional ultrasound images and the blood pressure measurements needs to be correlated in the method of Fig. 4. Rather, the blood pressure measurement is performed before the scans in the ultrasound procedure at S401 and consists of a systolic and a diastolic value such as 120/80. The systolic and diastolic values may be considered valid for all two-dimensional ultrasound image sequences.

At S407, probe pressure measurements are received. The probe pressure measurements may be pressure measurements of ultrasound probe pressure from when the pressure is variably applied to the subject. S407 may be optional in that some embodiments based on the method of Fig. 4 may be performed without obtaining pressure measurements from the ultrasound probe being applied over time. For example, a user may enter probe pressure as high or low responsive to guidance on the graphical user interface 181. When the pressure measurements from the ultrasound probe are applied at S407 for other embodiments, the measurements may be used in combination with arterial pressure measurements to determine advanced material properties. In embodiments in which the non-linear elastic relationship is determined based on pressure measurements of ultrasound probe pressure, the non-linear elastic relationship may be obtained based on a combination of the pressure measurements of ultrasound probe pressure as well as the arterial pressure measurements.

At S420, diameters of vasculature walls and differences in diameters are measured. Aortic diameter(s) and/or circumference(s) of vasculature walls and differences in aortic diameter(s) and/or circumference(s) are measured. Frames with maximum and/or minimum aortic diameter(s) and/or circumference(s) are measured. In some embodiments, S420 may include determining, within each acquired two-dimensional ultrasound image sequence, diastolic frames in which aortic diameter or circumference is minimum, and systolic frames in which aortic diameter is maximum.

At S430, changes in vasculature walls are determined. The changes in vasculature walls may be changes in the vasculature wall tissue between the two-dimensional ultrasound image sequences at the different times as the probe pressure is variably applied to the subject. The changes in the vasculature walls may be changes in aortic diameter or circumference variation in aortic walls of the abdominal aorta of the subject. The ultrasound system 100 in Fig. 1 or the arrangement in Fig. 2 may measure cyclic variations in aortic diameter or circumference of the vasculature walls.

As noted for S420, the method of Fig. 4 may also include determining within each acquired two-dimensional ultrasound image sequence, diastolic frames in which aortic diameter or circumference is minimum, and systolic frames in which aortic diameter is maximum. The measurements of aortic diameters or circumferences at S420 may include measuring a first aortic diameter or circumference at a first time corresponding to a diastolic frame and measuring a second aortic diameter or circumference at a second time corresponding to a systolic frame subsequent to the first time. The timing relationship of diastolic frames or systolic frames may be predetermined.

At S440, a non-linear elastic relationship is determined. The non-linear elastic relationship may be between strains in vasculature wall tissue captured in the two-dimensional ultrasound image sequences and varying stresses to which the vasculature wall tissue is subjected based on subject-specific blood pressure. The non-linear elastic relationship may be determined based on the blood pressure measurements and the changes in response of the vasculature wall tissue between the two-dimensional ultrasound image sequences as pressure is variably applied. A relationship between pressure and aortic diameter or circumference of the vasculature walls may be determined, so to extract constants of a non-linear hyperelastic material model based on the relationship to characterize the vasculature wall tissue. For example, a sensor in the ultrasound system 100 of Fig. 1 or a sensor provided with the ultrasound system 210 in Fig. 2 may measure probe pressure, and the non-linear elastic relationship may be determined also based on measurements of the probe pressure from the sensor. The sensor may comprise a blood pressure cuff, a direct arterial pressure sensor, or other elements used to measure arterial pressure.

As noted above, S407 may be optional in that and the remainder of the method of Fig. 4 may be performed based on the arterial pressure without obtaining probe pressure over time in some embodiments. When the probe pressure is available, the non-linear elastic relationship may be determined at S440 based on both the arterial pressure and the probe pressure over time.

At S450, an index indicating loss of elasticity is obtained. The method of Fig. 4 may include detecting as the aneurysmal state a loss of elasticity in the vasculature walls of the subject due to pathological state. The index may indicate the loss of elasticity based on measuring the cyclic variations in aortic diameter or circumference of the vasculature walls at S430.

At S460, an aneurysmal state is detected. The aneurysmal state is detected based on the non-linear elastic relationship and the changes in response of the vasculature wall tissue between the two-dimensional ultrasound image sequences as the pressure is variably applied. The aneurysmal state may also be detected based on the blood pressure measurements in combination with the non-linear elastic relationship and the changes in response of the vasculature wall tissue over time as the pressure is variably applied.

At S470, an output is generated and provided indicating the aneurysmal state. The output may be provided via the display 180 or smartphone A and/or smartphone B.

In a first set of embodiments for aneurysmal state detection, two or more B-mode ultrasound cineloops of the aorta may be acquired. The sequences are acquired at different values of probe pressure (PP), such as one at a relatively light probe pressure and one at a relatively firm probe pressure. The sequences are acquired on the same (axial) plane, which is the maximum diameter plane in the case of abdominal aorta aneurysm. The subject (or patient) may be directed to hold their breath for at least one full cardiac cycle during the acquisition, so that the image quality is adequate to detect the antero-posterior (AP) diameter of the abdominal aorta. In the first set of embodiments, arterial pressure measured via sphygmomanometer, or an equivalent method may be used to characterize strains in aorta wall tissue.

In a second set of embodiments which are an extension of the first set, material may be fully characterized by retrieving material parameters that define the mechanical behavior of the tissue in the full range of stresses and strains. The acquisition protocol in the second set of embodiments is the same as in the first. However, in the second set of embodiments, external probe pressure is also measured via a gel pad or a pressure sensor. Arterial pressure is again measured via sphygmomanometer or an equivalent method. For example, the controller 150 in Fig. 1 or smartphone A or smartphone B in Fig. 2 may receive measurements of arterial pressure and determine the non-linear elastic relationship based on the external probe pressure and also based on the measurements of the arterial pressure. Post processing in the second set of embodiments is the same as in the first, with the addition of fitting a polynomial curve to obtain the pressure-diameter relationship and then find the matching stress-strain relationship parameters via a previously built table.

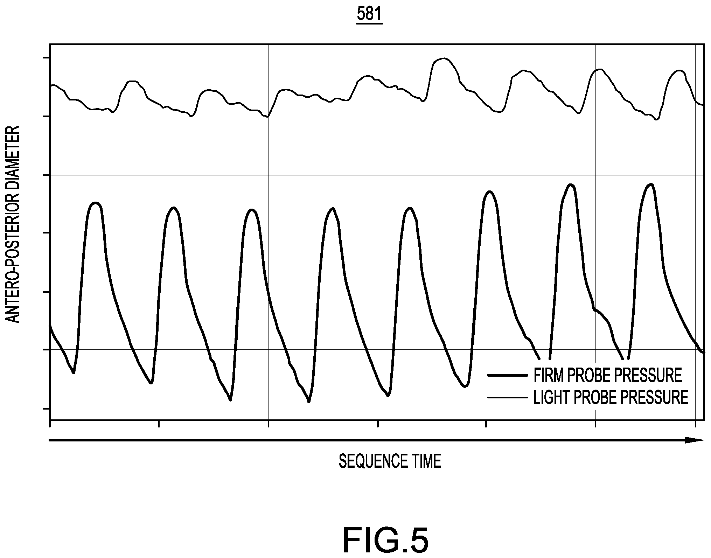

Fig. 5 illustrates antero-posterior diameter evolution in aneurysmal state detection, in accordance with a representative embodiment.

The evolution in Fig. 5 is from two successive scans acquired with light (top) and firm (bottom) probe pressure on the same subject, without probe motion. Combining two dynamic ultrasound acquisitions in two probe pressure conditions with a hyperelastic tissue model enables a correct estimation of arterial wall tissue material parameters. Firm probe pressure on the abdominal aortic aneurysm wall dynamics induces an increased wall distensibility in both abdominal aortic aneurysm subjects and in some healthy volunteers. This effect is not particularly intuitive but is a direct consequence of the abdominal aortic aneurysm tissue non-linear hyperelasticity as shown in Fig. 5 and can be used to estimate material parameters of the abdominal aortic aneurysm wall using change in aortic diameter variation in order to characterize the wall tissue as a non-linear hyperelastic material. The effect was discovered during one or more sessions of acquisitions on patients. The anticipated affect was that the amplitudes of diameter changes would be reduced with increased probe pressure, as is the case for the diameter itself. However, results showed an opposite or otherwise different effect. This enables obtaining reliable, reproducible, and comparable subject-specific indicators of loss of elasticity in the arterial wall due to pathological state. This also is clinically applicable, as it is directly based on clinically accessible subject-specific data and does not require any physical simulations.

The relationship is applied in the systems of FIG. 1, Fig. 2 and Fig. 3 using dedicated two-dimensional cine-loops acquisition protocol to acquire two dynamic ultrasound sequences with varying probe pressure. The ultrasound imaging workstation software/hardware for vascular imaging include the elements shown in Fig. 1, Fig. 2, and Fig. 3, including an ultrasound probe and a processor/memory combination to apply an image processing algorithm and an ultrasound speckle tracking algorithm. Measurement of the cyclic diameter variations within each of the two sequences is performed by software executed by a processor and allows to obtain an index indicating the loss of elasticity of the blood vessel wall.

In some embodiments, a dedicated two-dimensional ultrasound cine-loops acquisition protocol is used to acquire two dynamic ultrasound sequences with varying probe pressure and may involve using a gel-pad system between the subject and the ultrasound probe or a pressure sensor mounted on the ultrasound probe for the measurement of ultrasound probe pressure, to measure the cyclic diameter variations within each of the two sequences. A non-linear hyperelastic material model fitting algorithm may be applied. Additionally, arterial pressure may be measured using, for example, a sphygmomanometer or any other device for arterial pressure measurement. As a result, these embodiments allow to obtain, in addition to the loss of elasticity index, a full characterization of the blood vessel tissue properties.

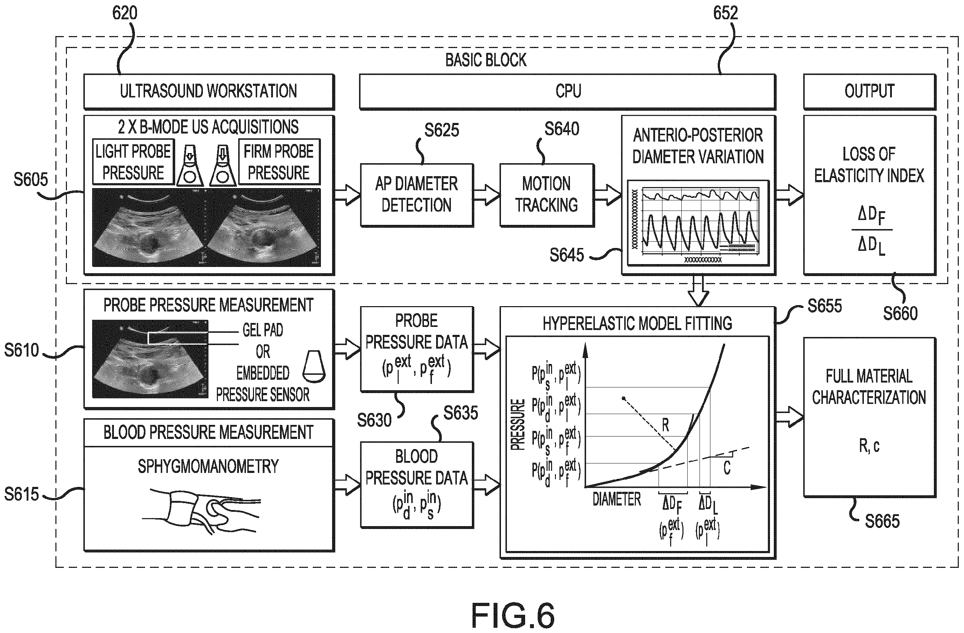

Fig. 6 illustrates a workflow for aneurysmal state detection, in accordance with a representative embodiment.

In Fig. 6, an ultrasound workstation 620 includes a central processing unit 652 (CPU). The ultrasound workstation 620 may correspond to the ultrasound base 120 in Fig. 1, and the central processing unit 652 may correspond to the processor 152 in Fig. 1. The ultrasound workstation 620 performs a process that includes acquiring two B-mode ultrasound sequences at different values of probe pressure (PP) at S605. The two sequences are acquired on the same (axial) plane, such as the plane with the maximum aortic diameter in the case of an aneurysmal abdominal aorta. The patient may be instructed to hold their breath for at least one full cardiac cycle during the acquisition. Subsequently, antero-posterior (AP) diameter is detected at S625, and a motion tracking algorithm is employed at S640 to retrieve the antero-posterior diameter variation throughout each sequence at S645. A speckle tracking algorithm may be used to track the arterial wall motion throughout the entire sequence or a sub-sequence of interest. The ratio between the diameter variation acquired with firm probe pressure and the one acquired with light probe pressure is determined as an output at S660 as an indicator of the loss of elasticity in the aneurysm. A mean (cyclic) antero-posterior (AP) diameter variation may be attributed to blood pressure and may be calculated for each sequence or sub-sequence of interest at S625. The extended version taught herein requires, in addition, the measurement of the probe pressure at S610 and the blood pressure of the subject at S615. Then, a pressure/diameter relationship is retrieved by interpolating the subject-specific values from the probe pressure data at S630 and from the blood pressure data at S635. From this curve, the constants of a non-linear hyperelastic material model (R = radius of curvature C= tangent modulus of elastin) can be extracted at S655 to characterize the full material at S665. The index for the loss of elasticity may be calculated as the ratio between the diameter variation at firm probe pressure and the diameter variation at light probe pressure.

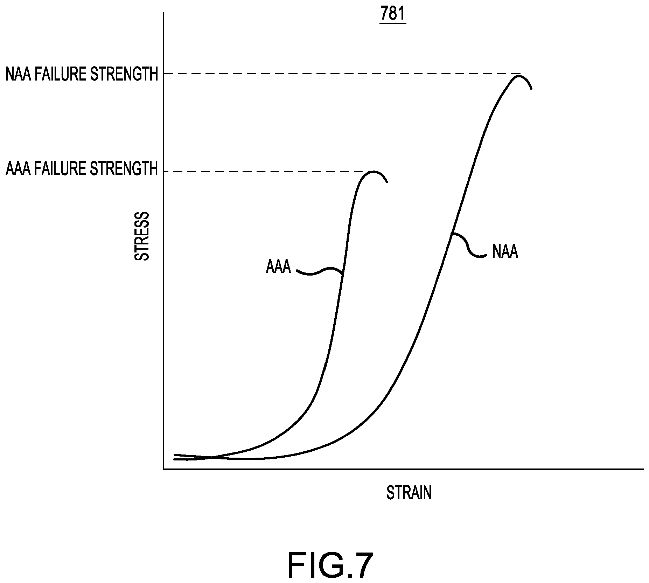

Fig. 7 illustrates a relationship between stress and strain in aneurysmal state detection, in accordance with a representative embodiment.

Fig. 7 shows a relationship between stress and strain for aneurysmatic and non-aneurysmatic abdominal aorta wall tissue. The AAA curve on the user interface 781 in Fig. 7 is for aneurysmatic abdominal aorta (AAA) wall tissue stress (vertical axis) relative to strain (horizontal axis). The NAA curve on the user interface 781 in Fig. 7 is for non-aneurysmatic abdominal aorta wall tissue stress (vertical axis) relative to strain (horizontal axis). As can be seen, the failure strength of aneurysmatic abdominal aorta wall tissue is lower and at a lower level of strain than the failure strength of non-aneurysmatic abdominal aorta wall tissue. The relatively lower failure strength of the aneurysmatic abdominal aorta wall tissue emphasizes the importance of an ability to dynamically characterize the effects of strains in vasculature wall tissue captured as taught herein.

Post-processing of sequences may be performed by a processor of a controller. The antero-posterior diameter may be detected either manually or automatically in all acquisitions. A speckle tracking algorithm may be used to track the arterial wall motion through the entire sequence or a portion of interest of the sequence. The (mean) cyclic antero-posterior diameter variation due to blood pressure may be calculated for each sequence or portion of interest of the sequence. A loss of elasticity index may be calculated as the ratio between the diameter variation at firm probe pressure and the diameter variation at light probe pressure. The obtained ratio Δ**D**sub**F**/Δ**D**sub**L** corresponds to the ratio between the tangent elastic moduli ***E***sub**L**/***E***sub**F**. As the elastin content in the vascular wall diminishes, the collagen fibers behavior is predominant, and therefore the obtained ratio should be close to 1 insofar as the tissue becomes more linear. Conversely, when the tissue elastin content is higher, the ratio value increases, capturing the non-linear hyperelastic behavior given by the combination of collagen and elastin. Therefore, this simple measure provides an indication of the degradation status of a subject's artery. The strain stress curves illustrating this mechanism are shown in Fig. 7.

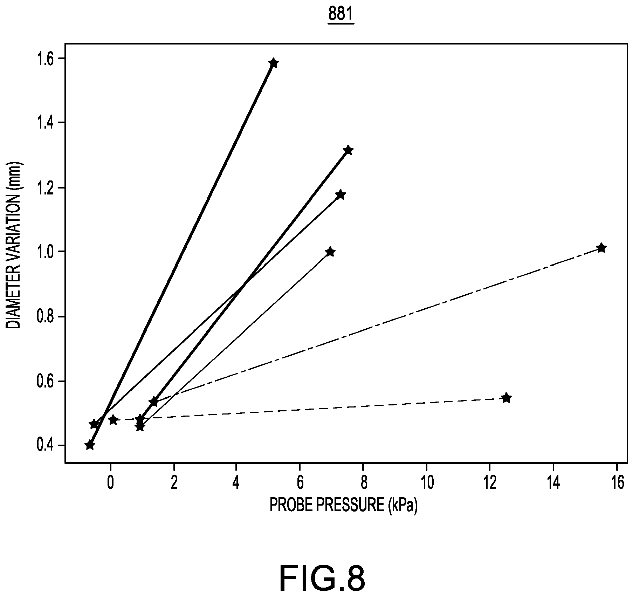

Fig. 8 illustrates antero-posterior diameter changes in three subjects in aneurysmal state detection, in accordance with a representative embodiment.

In Fig. 8, antero-posterior diameter change in three subjects is shown. Patient 1 has the two lines ending at the two highest points. Patient 2 has the two lines ending at the two second highest points. Patient 3 has the two lines ending at the two lowest points. The readings of diameter variation may be acquired with two probes and the coordinates may be set with two added values of probe pressure. The probe pressure values may be measured with a gel-pad, for example. The values of diameter change and probe pressure in Fig. 8 are for abdominal aortic aneurysm (abdominal aortic aneurysm) subjects. Such subjects may follow up on interventions at clinics as, for example, a functional complement to two-dimensional ultrasound abdominal aortic aneurysm measurements using a tool to measure strain or as an additional to an abdominal aortic aneurysm model. The teachings herein may be integrated with two-dimensional B-mode ultrasound to monitor abdominal aortic aneurysm subjects following existing clinical guidelines. An operator may be instructed to perform two extra acquisitions, such as between 2 and 10 seconds each. A pressure sensor may be added to the ultrasound probe and the operator may be instructed to acquire patient brachial pressure. In Fig. 8, feasibility of measuring strain to detect aneurysmal status was tested for the three subjects in Fig. 8 with antero-posterior diameters of 47.5 mm, 35 mm, and 30 mm. The loss of elasticity coefficient computed for each subject results in ratio **ΔDF** values of 0.37, 0.5 and 0.25 **ΔDL** respectively. The values of diameter change, and probe pressure are reported in Fig. 8. As shown, significant differences exist among the three subjects. Stratification of subjects using such strain measurements may provide benefits insofar as currently no clear relationship between the aneurysm size and the calculated index can be found. Insofar as reduced strain may relate to a higher rate of rupture, the diagnostic effect of stratification may be used to reduce risk for some patients.

Fig. 9 illustrates a computer system, on which a method for aneurysmal state detection is implemented, in accordance with another representative embodiment.

Referring to Fig. 9, the computer system 900 includes a set of software instructions that can be executed to cause the computer system 900 to perform any of the methods or computer-based functions disclosed herein. The computer system 900 may operate as a standalone device or may be connected, for example, using a network 901, to other computer systems or peripheral devices. In embodiments, a computer system 900 performs logical processing based on digital signals received via an analog-to-digital converter.

In a networked deployment, the computer system 900 operates in the capacity of a server or as a client user computer in a server-client user network environment, or as a peer computer system in a peer-to-peer (or distributed) network environment. The computer system 900 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 900 can be incorporated as or in a device that in turn is in an integrated system that includes additional devices. In an embodiment, the computer system 900 can be implemented using electronic devices that provide voice, video, or data communication. Further, while the computer system 900 is illustrated in the singular, the term "system" shall also be taken to include any collection of systems or sub-systems that individually or jointly execute a set, or multiple sets, of software instructions to perform one or more computer functions.

As illustrated in Fig. 9, the computer system 900 includes a processor 910. The processor 910 may be considered a representative example of a processor of a controller and executes instructions to implement some or all aspects of methods and processes described herein. The processor 910 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 910 is an article of manufacture and/or a machine component. The processor 910 is configured to execute software instructions to perform functions as described in the various embodiments herein. The processor 910 may be a general-purpose processor or may be part of an application specific integrated circuit (ASIC). The processor 910 may also be a microprocessor, a microcomputer, a processor chip, a controller, a microcontroller, a digital signal processor (DSP), a state machine, or a programmable logic device. The processor 910 may also be a logical circuit, including a programmable gate array (PGA), such as a field programmable gate array (FPGA), or another type of circuit that includes discrete gate and/or transistor logic. The processor 910 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 900 further includes a main memory 920 and a static memory 930, where memories in the computer system 900 communicate with each other and the processor 910 via a bus 908. Either or both of the main memory 920 and the static memory 930 may be considered representative examples of a memory of a controller, and store instructions used to implement some, or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 920 and the static memory 930 are articles of manufacture and/or machine components. The main memory 920 and the static memory 930 are computer-readable mediums from which data and executable software instructions can be read by a computer (e.g., the processor 910). Each of the main memory 920 and the static memory 930 may be implemented as one or more of random-access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 900 further includes a video display unit 950, such as a liquid crystal display (LCD), an organic light emitting diode (OLED), a flat panel display, a solid-state display, or a cathode ray tube (CRT), for example. Additionally, the computer system 900 includes an input device 960, such as a keyboard/virtual keyboard or touch-sensitive input screen or speech input with speech recognition, and a cursor control device 970, such as a mouse or touch-sensitive input screen or pad. The computer system 900 also optionally includes a disk drive unit 980, a signal generation device 990, such as a speaker or remote control, and/or a network interface device 940.

In an embodiment, as depicted in Fig. 9, the disk drive unit 980 includes a non-transitory computer-readable medium 982 in which one or more sets of software instructions 984 (software) are embedded. The sets of software instructions 984 are read from the non-transitory computer-readable medium 982 to be executed by the processor 910. Further, the software instructions 984, when executed by the processor 910, perform one or more steps of the methods and processes as described herein. In an embodiment, the software instructions 984 reside all or in part within the main memory 920, the static memory 930 and/or the processor 910 during execution by the computer system 900. Further, the non-transitory computer-readable medium 982 may include software instructions 984 or receive and execute software instructions 984 responsive to a propagated signal, so that a device connected to a network 901 communicates voice, video, or data over the network 901. The software instructions 984 may be transmitted or received over the network 901 via the network interface device 940.

The computer program product may be software available for download from a server, e.g., via the internet. Alternatively, the computer program product may be a suitable (non-transitory) computer readable medium on which the instructions are stored, such as an optical storage medium or a solid-state medium, which may or may not be supplied together with or as part of other hardware.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, aneurysmal state detection enables improved accuracy when detecting aneurysmal states of vasculature wall tissue. A non-linear elastic relationship between the strains in the tissue and the stresses to which the tissue subjected may be extracted by characterizing aneurysmal tissue via probe pressure-calibrated ultrasound imaging. Arterial tissue mechanics may be characterized in vivo by means of two-dimensional ultrasound acquisitions with varying probe pressure. Updating the wall stiffness measurements to rely on a non-linear hyperelastic material model, the values of tangent stiffness modulus can reflect the external probe pressure, and this may result in more accurate measurements. Accounting for the non-linearity of mechanical behavior of blood vessels may result in improved accuracy when detecting aneurysmal states of vasculature wall tissue.

Although aneurysmal state detection has been described with reference to several exemplary embodiments, it is understood that the words that have been used are words of description and illustration, rather than words of limitation. Changes may be made within the purview of the appended claims, as presently stated, and as amended, without departing from the scope and spirit of aneurysmal state detection in its aspects. Although aneurysmal state detection has been described with reference to particular means, materials, and embodiments, aneurysmal state detection is not intended to be limited to the particulars disclosed; rather aneurysmal state detection extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The abstract is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. A method of determining an aneurysmal state of vasculature wall tissue, the method comprising:
receiving (S403) time-resolved two-dimensional ultrasound image sequences of the vasculature wall tissue captured at different times as pressure is variably applied to a subject of the two-dimensional ultrasound image sequences;
receiving (S405) blood pressure measurements of the subject;
determining (S430) changes in response of the vasculature wall tissue between the two-dimensional ultrasound image sequences at the different times as the pressure is variably applied to the subject;
determining (S440), based on the blood pressure measurements and the changes in response of the vasculature wall tissue between the two-dimensional ultrasound image sequences as the pressure is variably applied, a non-linear elastic relationship between strains in vasculature wall tissue captured in the two-dimensional ultrasound image sequences and varying stresses to which the vasculature wall tissue is subjected based on subject-specific blood pressure; and
detecting (S460) the aneurysmal state of the vasculature wall tissue based on the non-linear elastic relationship and the changes in response of the vasculature wall tissue between the two-dimensional ultrasound image sequences as the pressure is variably applied.

2. The method of claim 1, wherein the vasculature walls comprise aortic walls of an abdominal aorta of the subject, and the changes in the vasculature walls comprise changes in aortic diameter or circumference variation in the aortic walls.

3. The method of claim 1 or 2, further comprising:
measuring (S420) a first aortic diameter or circumference of the vasculature walls at a first time;
measuring (S420) a second aortic diameter or circumference of the vasculature walls at a second time different to the first time;
determining (S420) a difference between the first aortic diameter or circumference and the second aortic diameter or circumference; and
determining (S430) the changes in response of the vasculature wall tissue as the pressure is variably applied based on the difference between the first aortic diameter or circumference and the second aortic diameter or circumference.

4. The method of claim 3, further comprising:
determining (S420) within each acquired two-dimensional ultrasound image sequence, diastolic frames in which aortic diameter or circumference is minimum, corresponding to the first time, and systolic frames in which aortic diameter is maximum, corresponding to the second time.

5. The method of any one of claims 1 to 4, wherein the two-dimensional ultrasound image sequences captured at different times are captured in a single cineloop.

6. The method of any one of claims 1 to 5, further comprising:
determining the non-linear elastic relationship between strains in vasculature wall tissue captured in the two-dimensional ultrasound image sequences and varying stresses to which the vasculature wall tissue is subjected based on a combination of externally applied probe pressure (S407) variably applied to the subject and the subject-specific blood pressure (S405); and
detecting as the aneurysmal state a loss of elasticity in the vasculature walls of the subject due to pathological state.

7. The method of claim 6, further comprising:
measuring (S420) cyclic variations in aortic diameter or circumference of the vasculature walls; and
obtaining (S450) an index indicating the loss of elasticity based on measuring the cyclic variations in aortic diameter or circumference of the vasculature walls.

8. The method of claim 7, wherein the two-dimensional ultrasound image sequences are captured at different times in two separate cineloops, and
the cyclic variations in aortic diameter or circumference of the vasculature walls are measured within each of the two separate cineloops.

9. The method of any one of claims 1 to 8, further comprising:
receiving (S405) measurements of arterial pressure; and
determining the non-linear elastic relationship also based on measurements of the arterial pressure.

10. The method of any one of claims 1 to 9, further comprising:
determining (S440) a relationship between pressure and aortic diameter or circumference of the vasculature walls;
extracting (S440) constants of a non-linear hyperelastic material model based on the relationship to characterize the vasculature wall tissue.

11. A computer program product comprising instructions for enabling a processor (152, 910) to carry out the method according to any one of claims 1 to 10.

12. An ultrasound system (100) comprising:
an ultrasound probe (110); and
a processor (152, 910) in communication with the ultrasound probe, the processor being configured to carry out the method according to any one of claims 1 to 10.

13. The system of claim 12, further comprising:
a sensor for measuring arterial pressure.
